# EUROPEAN PATENT APPLICATION

(11) **EP 4 606 340 A1**
(43) Date of publication of application: **27.08.2025**
(21) Application number: 23880215.1
(22) Date of filing: 18.10.2023
(51) Int. Cl.: A61B 34/30, A61B 34/37, A61B 90/00, A61B 17/00

(54) **SURGICAL TOOL GUIDE CONNECTED TO SURGICAL TOOL CONTROL DEVICE**

(30) Priority: 20.10.2022 KR 20220136004; 03.04.2023 KR 20230043642
(71) Applicant: LN Robotics Inc., Seoul 05505 (KR)
(72) Inventor: WON, Jong Seok, Seoul 06226 (KR)
(74) Representative: Lavoix
(86) International application number: PCT/KR2023/016118
(87) International publication number: WO 2024/085638

(57) **Abstract**

In one embodiment, a surgical tool guide connected to a surgical tool control device may comprise: a bottom guide including a plurality of channels of which the tops are open such that surgical tools can be placed therein; and a top guide connected to the top side of the bottom guide such that the top of at least a portion of each channel is closed.

## Description

### TECHNICAL FIELD

The following embodiment relates to a medical instrument guide connected to a medical instrument control device.

### BACKGROUND ART

In conventional percutaneous coronary intervention (PCI), a practitioner bears the risk of continuous radiation exposure, significant time and costs are required to train a skilled doctor for stable treatment, and it is difficult to commonly provide high-level medical services because of gaps among practitioners, regions, and hospitals in terms of treatment completion level. To compensate for these shortcomings, an intervention procedure assistant robot is introduced. For example, the intervention procedure assistant robot may be configured to move forward, move backward, or rotate a medical instrument when a user inputs a command.

The above description is information the inventor(s) acquired during the course of conceiving the present disclosure, or already possessed at the time, and is not necessarily art publicly known before the present application was filed.

Korean Patent No. 10-2019-0121928 (published on October 29, 2019) discloses a driving device for a medical robot and a medical robot.

### DISCLOSURE OF THE INVENTION

### TECHNICAL GOALS

A goal of an embodiment is to provide a medical instrument guide for stably guiding a path of a plurality of medical instruments in a medical instrument control device that independently controls the plurality of medical instruments for complex percutaneous intervention (PCI).

A goal of an embodiment is to provide a medical instrument guide for easily placing a medical instrument on each channel.

### TECHNICAL SOLUTIONS

In an embodiment, a medical instrument guide connected to a medical instrument control device may include a lower guide in which a plurality of channels with open upper portions is formed to dispose a medical instrument thereon, and an upper guide connected to an upper side of the lower guide to close an upper portion of at least a section of each of the channels.

The lower guide may include a lower base body, a plurality of roller covers positioned on a distal of the lower base body to cover at least a portion of a plurality of roller modules provided in the medical instrument control device from an upper portion, and a plurality of main valleys forming at least a section of the channel and extending downward between the plurality of roller covers to be positioned between each two of the plurality of roller modules.

The upper guide may include an upper base body placed on an upper side of the plurality of roller covers, and a plurality of legs extending downward from a lower portion of the upper base body to be inserted into each of the plurality of main valleys.

While the upper guide is connected to the lower guide, the plurality of legs may close the upper portion of each of the channels formed on the plurality of main valleys.

Each of the main valleys may include a valley body forming at least a section of the channel, and a support bump protruding in a distal direction or a proximal direction at a lower end of the valley body.

Each of the legs may include a blade that is inserted into the valley body and closes the upper portion of the channel formed on the valley body, a pillar formed to have a width that is wider than the blade at a distal end or a proximal end of the blade, and a toe formed in a shape corresponding to the support bump at a lower end of the pillar.

The support bump and the toe each may include a central space for forming the channel, and inclined walls formed on both sides of the central space and inclined downward from a center of the central space toward the both sides.

The lower guide may further include a plurality of expanded spaces formed with a width that is greater than the main valley between the roller cover and the lower base body on a proximal side of each of the plurality of main valleys.

The lower guide may further include a plurality of recessed valleys recessed from the lower base body to form the channel together with the plurality of main valleys.

The lower guide may further include a plurality of expansion grooves recessed from the lower base body to have a wider width than the recessed valley at a proximal end of each of the plurality of recessed valleys.

The lower guide may further include a holder disposition hole penetrating the lower base body at a position of at least a portion of the plurality of recessed valleys to dispose a holder for holding a medical instrument positioned on the recessed valley.

The lower guide further may include an additional support bump protruding from a distal end of at least a portion of the plurality of recessed valleys in a distal direction, and the upper guide further may include an additional leg extending downward from a lower portion of the upper base body to be placed on the additional support bump.

Each of the plurality of recessed valleys may include a first recessed space of which a width narrows toward a lower side, and a second recessed space of which a width remains the same at a lower side compared to the first recessed space.

At least one magnet may be disposed on the plurality of roller covers and at least one magnet may be disposed on the upper base body to couple the lower guide and the upper guide to each other by a magnetic force.

The upper guide may further include a detachment assist formed by cutting a portion of a side surface and a bottom surface of the upper base body to elastically rotate relative to the upper base body to help decoupling of magnetic coupling between the lower guide and the upper guide.

### EFFECTS OF THE INVENTION

According to a medical instrument guide in an embodiment, a path of a plurality of medical instruments gripped by a medical instrument control device may be stably guided.

According to a medical instrument guide in an embodiment, convenience in placing a medical instrument on each channel may be improved.

The effects of the medical instrument guide are not limited to the above-mentioned effects, and other unmentioned effects can be clearly understood from the above description by those having ordinary skill in the technical field to which the present disclosure pertains.

### BRIEF DESCRIPTION OF DRAWINGS

FIG. 1 is a perspective view of a medical instrument control device according to an embodiment.
FIG. 2 is a plan view of a medical instrument control device according to an embodiment.
FIG. 3 is an exemplary usage state view of a medical instrument control device according to an embodiment.
FIG. 4 is a front view of a portion of a roller module of a medical instrument control device according to an embodiment.
FIG. 5 is a perspective view of a medical instrument guide according to an embodiment.
FIG. 6 is a perspective view of a lower guide according to an embodiment.
FIG. 7 is a bottom perspective view of a lower guide according to an embodiment.
FIG. 8 is a plan view of a lower guide according to an embodiment.
FIG. 9 is a perspective view of a main valley according to an embodiment.
FIG. 10 is a cross-sectional view of a recessed valley according to an embodiment.
FIG. 11 is a perspective view of an upper guide according to an embodiment.
FIG. 12 is a bottom perspective view of an upper guide according to an embodiment.
FIG. 13 is a bottom view of an upper guide according to an embodiment.
FIG. 14 is a perspective view of a leg according to an embodiment.
FIG. 15 is an enlarged view of portion A of FIG. 5.
FIG. 16 is a cross-sectional view of portion B-B of FIG. 15.

### BEST MODE FOR CARRYING OUT THE INVENTION

This patent application claims the benefit of patent application No. 10-2022-0136004, filed on October 20, 2022, the entire disclosure of which is incorporated herein by reference for all purposes.

Hereinafter, embodiments will be described in detail with reference to the accompanying drawings. However, various alterations and modifications may be made to the embodiments. Here, the embodiments are not meant to be limited by the descriptions of the present disclosure. The embodiments should be understood to include all changes, equivalents, and replacements within the idea and the technical scope of the disclosure.

The terminology used herein is for the purpose of describing particular example embodiments only and is not to be limiting of the example embodiments. As used herein, the singular forms "a," "an," and "the" are intended to include the plural forms as well, unless the context clearly indicates otherwise. It should be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like constituent elements and a repeated description related thereto will be omitted. In the description of embodiments, detailed description of well-known related structures or functions will be omitted when it is deemed that such description will cause ambiguous interpretation of the present disclosure.

Also, in the description of the components, terms such as first, second, A, B, (a), (b) or the like may be used herein when describing components of the present disclosure. These terms are used only for the purpose of discriminating one constituent element from another constituent element, and the nature, the sequences, or the orders of the constituent elements are not limited by the terms. It should be noted that if one component is described as being "connected," "coupled" or "joined" to another component, the former may be directly "connected," "coupled," and "joined" to the latter or "connected", "coupled", and "joined" to the latter via another component.

The same name may be used to describe an element included in the embodiments described above and an element having a common function. Unless otherwise mentioned, the description of one embodiment may be applicable to other embodiments. Thus, duplicated description is omitted for conciseness.

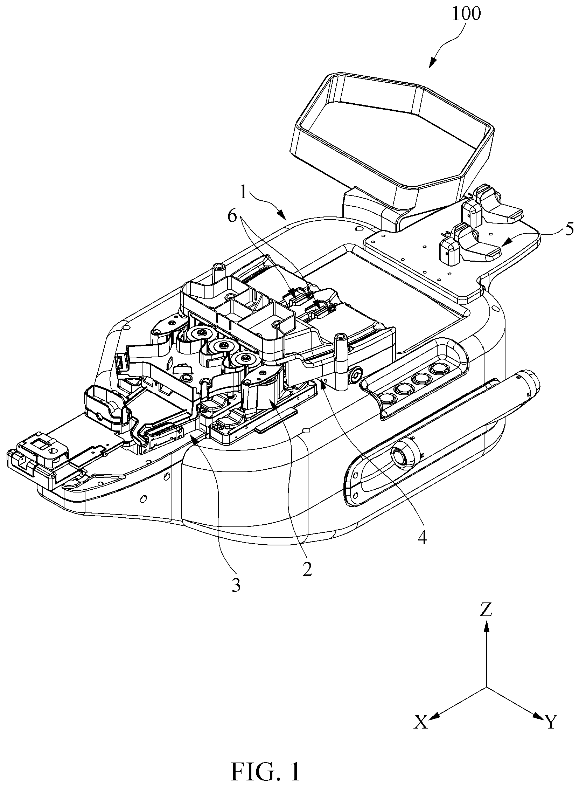

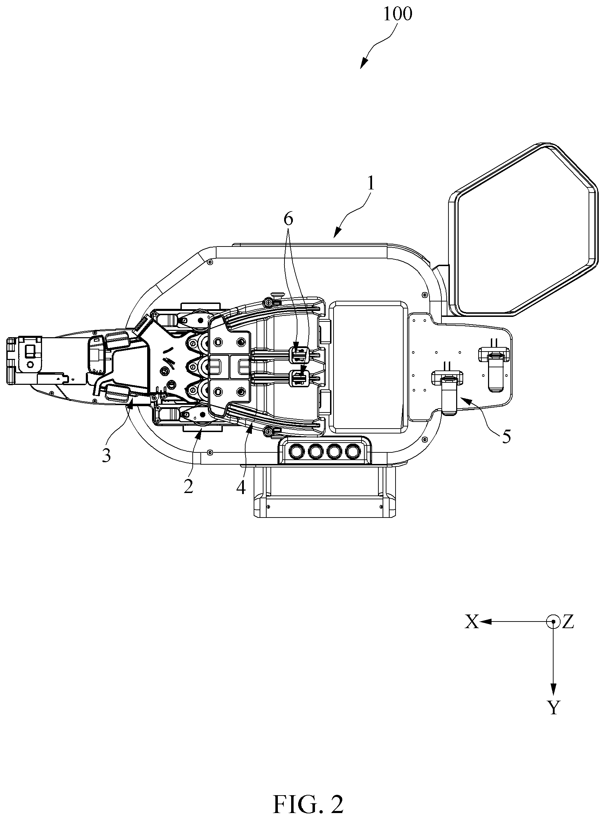

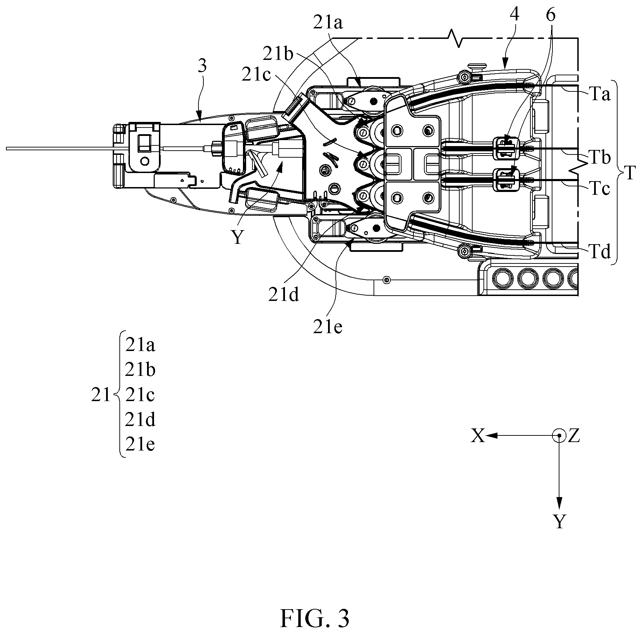

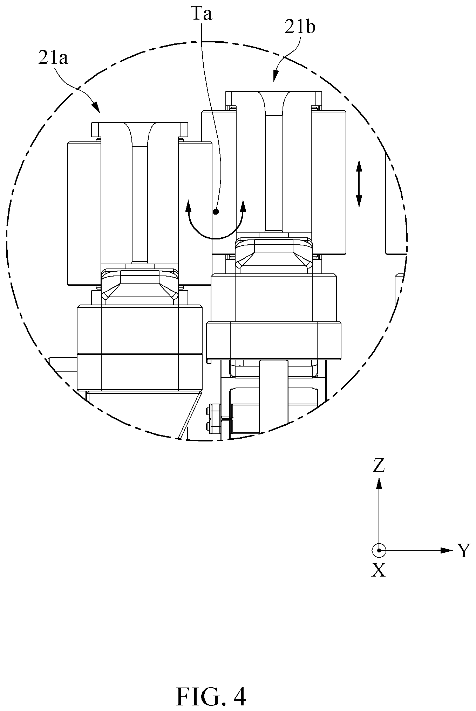

FIG. 1 is a perspective view of a medical instrument control device according to an embodiment. FIG. 2 is a plan view of a medical instrument control device according to an embodiment. FIG. 3 is an exemplary usage state view of a medical instrument control device according to an embodiment. FIG. 4 is a front view of a portion of a roller module of a medical instrument control device according to an embodiment.

Referring to FIGS. 1 to 4, a medical instrument control device 100 may independently control at least one medical instrument T. The medical instrument control device 100 may grip or release the medical instrument T between roller modules 21 described below by horizontal (e.g., the y direction) movement of the roller module 21. The medical instrument control device 100 may implement forward/backward movement and/or rotation of the medical instrument T by rotation and/or vertical (e.g., the z direction) movement of the roller module 21. The medical instrument control device 100 may be used for, for example, percutaneous coronary intervention (PCI). However, this is an example and the purpose of the medical instrument control device 100 is not limited thereto. The medical instrument T may refer to a medical instrument having a longitudinal direction. For example, the medical instrument T may include at least one of a guide wire, a balloon catheter, and a guide catheter having a longitudinal direction. However, this is an example and the type of the medical instrument T is not limited thereto.

Hereinafter, in the description of the medical instrument control device 100, it may be construed that proximal may refer to a side in the -x direction and distal may refer to a side in the +x direction.

The medical instrument control device 100 according to an embodiment may include a housing 1, a driving assembly 2, a connector mounter 3, a medical instrument guide 4, a rear holder 5, and a front holder 6.

In an embodiment, the housing 1 may form an exterior of the medical instrument control device 100. The housing 1 may provide an area where the driving assembly 2, the connector mounter 3, the medical instrument guide 4, the rear holder 5, and/or the front holder 6 are supported. For example, the housing 1 may be connected to a robotic arm of a slave base. The medical instrument control device 100 may function as an end effector connected to the robotic arm of the slave base. In the housing 1, a handle and/or a switch for adjusting a position of the housing 1 and/or tilting the housing 1 may be provided. In the housing 1, a display panel for conveying information on a state of the device and/or how to use the device to the user may be provided.

In an embodiment, the driving assembly 2 may grip or release the medical instrument T. The driving assembly 2 may move or rotate the gripped medical instrument T in a longitudinal direction.

In an embodiment, the driving assembly 2 may include a plurality of roller modules 21. At least a pair of roller modules 21 may be provided. For example, as shown in the drawing, the roller module 21 may include a first roller module 21a, a second roller module 21b, a third roller module 21c, a fourth roller module 21d, and a fifth roller module 21e. The plurality of roller modules 21 may be arranged parallel to each other. For example, the driving assembly 2 may independently control four medical instruments Ta, Tb, Tc, and Td by five roller modules 21a, 21b, 21c, 21d, and 21e. However, this is an example, and the number of roller modules 21 is not limited thereto.

In an embodiment, the medical instrument T may be gripped between two adjacent roller modules 21. For this, at least one roller module 21 may horizontally move toward the other roller module 21. For example, the second roller module 21b may horizontally move toward the first roller module 21a such that the first medical instrument Ta is gripped between the first roller module 21a and the second roller module 21b. In this case, the second medical instrument Tb positioned between the second roller module 21b and the third roller module 21c may be released. Additionally, on the other hand, the second roller module 21b may horizontally move toward the third roller module 21c such that the second medical instrument Tb is gripped between the second roller module 21b and the third roller module 21c. In this case, the first medical instrument Ta positioned between the first roller module 21a and the second roller module 21b may be released.

Similarly, the fourth roller module 21d may horizontally move toward the third roller module 21c such that the third medical instrument Tc is gripped between the third roller module 21c and the fourth roller module 21d. In this case, the fourth medical instrument Td positioned between the fourth roller module 21d and the fifth roller module 21e may be released. Additionally, on the other hand, the fourth roller module 21d may horizontally move toward the fifth roller module 21e such that the fourth medical instrument Td is gripped between the fourth roller module 21d and the fifth roller module 21e. In this case, the third medical instrument Tc positioned between the third roller module 21c and the fourth roller module 21d may be released.

In an embodiment, the driving assembly 2 may implement forward/backward movement and/or rotation of the medical instrument T by rotation and/or vertical movement of the roller module 21. For example, while the first roller module 21a and the second roller module 21b are positioned adjacent to each other and grip the first medical instrument Ta therebetween, the gripped first medical instrument Ta may move forward or backward in the longitudinal direction by rotating the first roller module 21a and the second roller module 21b in one direction or other directions. In addition, as shown in FIG. 4, while the first roller module 21a and the second roller module 21b are positioned adjacent to each other and grip the first medical instrument Ta therebetween, the gripped first medical instrument Ta may rotate by moving at least one of the roller modules 21a and 21b in the vertical direction.

In an embodiment, the connector mounter 3 may be connected to the housing 1 to be positioned distal (e.g., a side in the +x direction) of the housing 1. The connector mounter 3 may be attached to and detached from the housing 1. The connector mounter 3 may be a consumable and replaceable. The connector mounter 3 may include, for example, a polycarbonate material. However, this is an example, and the material of connector mounter 3 is not limited thereto.

In an embodiment, the connector mounter 3 may hold a medical instrument connector Y. For example, the medical instrument connector Y may be a component for collecting at least one medical instrument T and guiding a path of the at least one medical instrument T. For example, the medical instrument connector Y may include a Y connector. However, this is an example and the type of the medical instrument connector Y is not limited thereto. The connector mounter 3 may guide a path of the medical instrument T passing through the medical instrument connector Y.

In an embodiment, the medical instrument guide 4 may be connected to the housing 1 to be positioned proximal (e.g., a side in the -x direction) compared to the connector mounter 3. The medical instrument guide 4 may be attached to and detached from the housing 1. The medical instrument guide 4 may be a consumable and replaceable. The medical instrument guide 4 may include, for example, a polycarbonate material. However, this is an example and the material of the medical instrument guide 4 is not limited thereto.

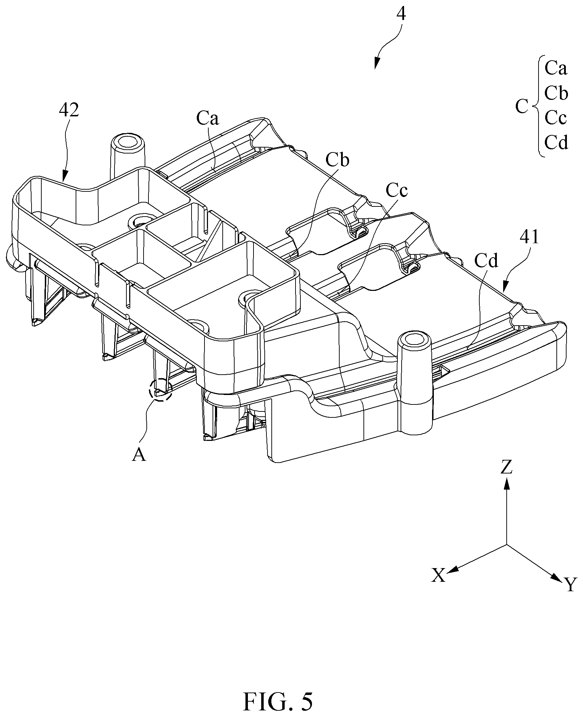

In an embodiment, the medical instrument guide 4 may guide a path of the medical instrument T gripped by the driving assembly 2. For example, the medical instrument guide 4 may be connected to the housing 1 to cover at least a portion (e.g., a proximal portion) of the driving assembly 2. The medical instrument guide 4 may include at least one channel (e.g., a channel C of FIG. 5) to insert and dispose the medical instrument T therein. Each channel C may be formed between two adjacent roller modules 21 of the driving assembly 2.

In an embodiment, the rear holder 5 may be connected to the housing 1 to be positioned proximal (e.g., the side in the -x direction) of the housing 1. The rear holder 5 may hold a position of the medical instrument T. For example, the rear holder 5 may include a clamp structure. For example, one rear holder 5 or a plurality of rear holders 5 may be provided. The rear holder 5 may be a consumable and replaceable. The rear holder 5 may include, for example, a polycarbonate material. However, this is an example, and the material of the rear holder 5 is not limited thereto.

In an embodiment, the front holder 6 may be connected to the housing 1 to be positioned distal (e.g., the +x direction) compared to the rear holder 5. For example, the front holder 6 may be positioned by penetrating the medical instrument guide 4. The front holder 6 may hold a position of the medical instrument T. For example, the front holder 6 may include a clamp structure. For example, one front holder 6 or a plurality of front holders 6 may be provided. The front holder 6 may be a consumable and replaceable. The front holder 6 may include, for example, a polycarbonate material. However, this is an example and the material of the front holder 6 is not limited thereto.

In an embodiment, the connector mounter 3, the medical instrument guide 4, the rear holder 5, and/or the front holder 6 may be sealed after sterilization and may be provided as disposable products. After the connector mounter 3, the medical instrument guide 4, the rear holder 5, and/or the front holder 6 are opened on site for each procedure and are coupled to the housing 1, the connector mounter 3, the medical instrument guide 4, the rear holder 5, and/or the front holder 6 may hold the plurality of medical instruments T or may guide the path. When the procedure is terminated, the connector mounter 3, the medical instrument guide 4, the rear holder 5, and/or the front holder 6 may be removed from the housing 1 and may be discarded.

FIG. 5 is a perspective view of a medical instrument guide according to an embodiment.

In an embodiment, referring to FIGS. 1 to 5, the medical instrument guide 4 may include a lower guide 41 and an upper guide 42. The lower guide 41 may be connected to the medical instrument control device 100. For example, the lower guide 41 may be directly or indirectly connected to the housing 1. A plurality of channels C of which upper portions are open (e.g., a portion in the +z direction) to dispose the medical instrument T may be formed on the lower guide 41. The channel C may be formed by continuously or discontinuously extending from a distal end (e.g., an end in the +x direction) of the lower guide 41 to a proximal end (e.g., an end in the -x direction). While the medical instrument T is placed on the channel C of the lower guide 41, the upper guide 42 may be connected to an upper side (e.g., the side in the +z direction) of the lower guide 41. The upper guide 42 may be attached to and detached from the lower guide 41. For example, the upper guide 42 may be attached to and detached from the lower guide 41 by a magnetic force. While the upper guide 42 is connected to the upper side (e.g., the side in the +z direction) of the lower guide 41, an upper portion (e.g., the portion in the +z direction) of at least some section of the channel C formed on the lower guide 41 may be closed by the upper guide 42.

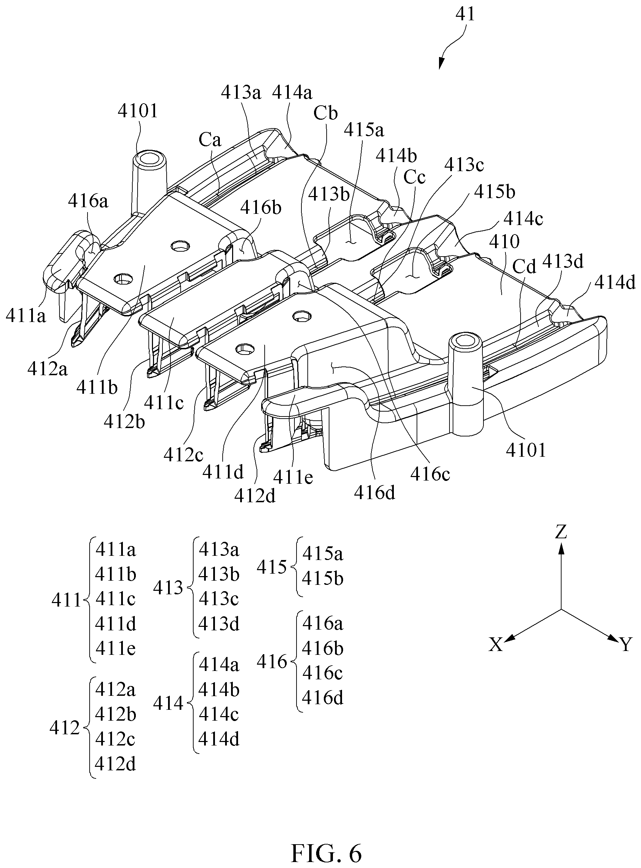

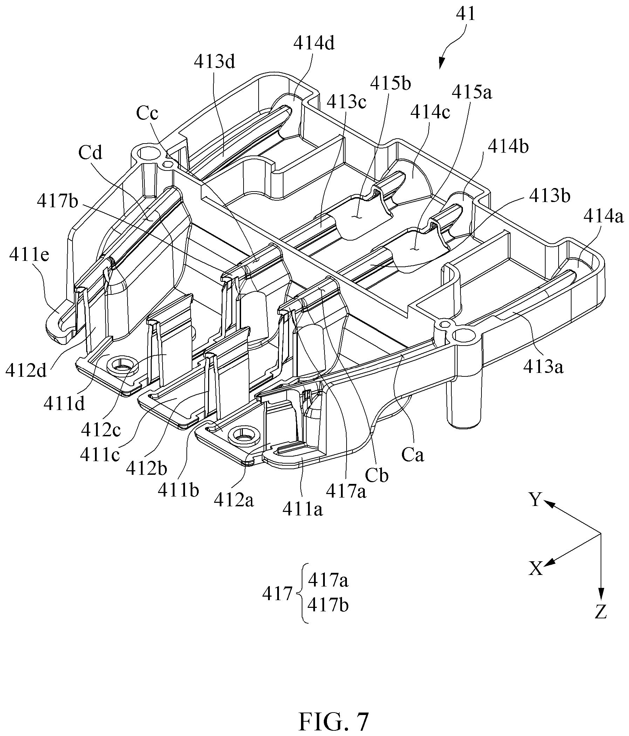

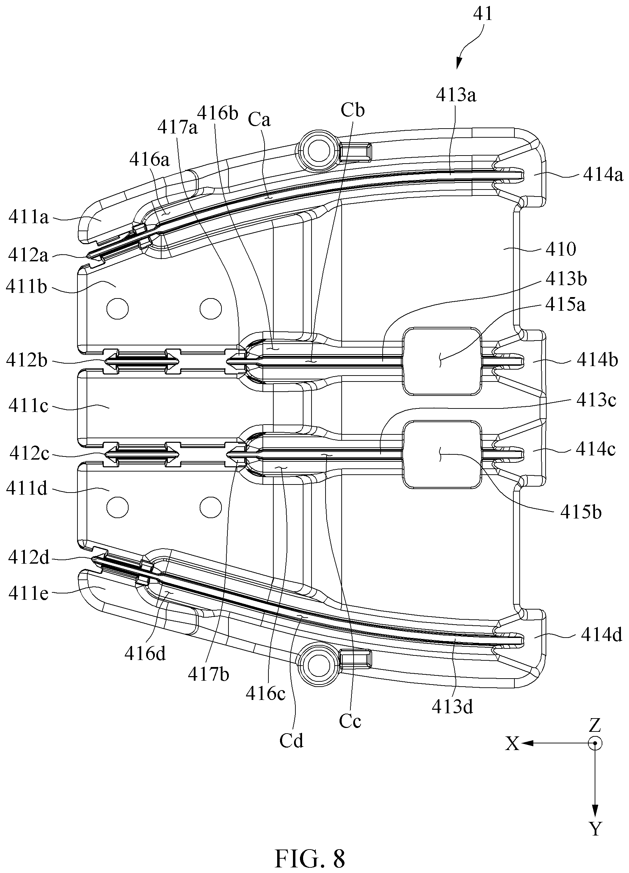

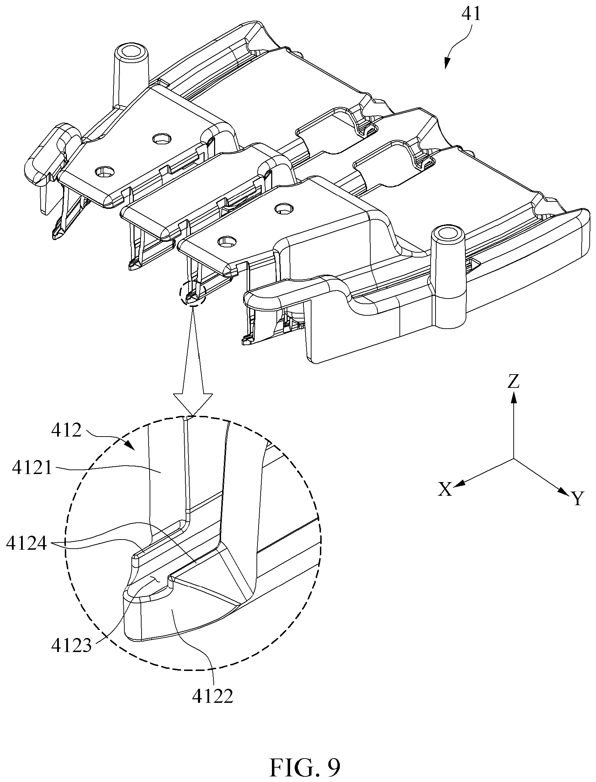

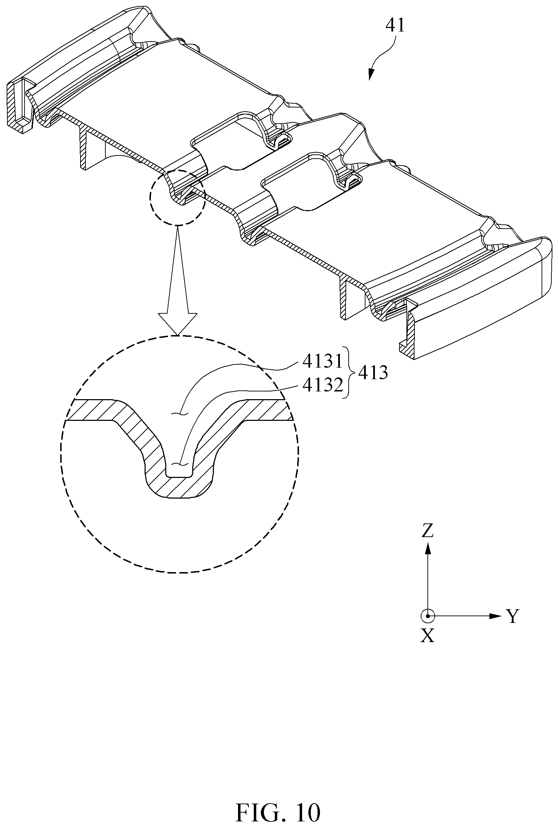

FIG. 6 is a perspective view of a lower guide according to an embodiment. FIG. 7 is a bottom perspective view of a lower guide according to an embodiment. FIG. 8 is a plan view of a lower guide according to an embodiment. FIG. 9 is a perspective view of a main valley according to an embodiment. FIG. 10 is a cross-sectional view of a recessed valley according to an embodiment.

Hereinafter, the lower guide 41 is described with reference to FIGS. 1 to 10. Hereinafter, in the description of the lower guide 41, it may be construed that proximal may refer to a side in the -x direction and distal may refer to a side in the +x direction.

In an embodiment, the lower guide 41 may include a lower base body 410, a roller cover 411, a main valley 412, a recessed valley 413, an expansion groove 414, a holder disposition hole 415, an expanded spaced 416, and an additional support bump 417.

In an embodiment, the lower base body 410 may form a base of the lower guide 41. The lower base body 410 may be indirectly or directly detachably connected to the housing 1. For example, a connecting structure 4101 for connecting to the housing 1 may be formed on the lower base body 410. For example, the connecting structure 4101 may have a structure to receive a protrusion positioned in the housing 1. However, this is an example and the shape, position, and/or number of the connecting structures 4101 are not limited thereto. The lower guide 41 may be detachably connected to the housing 1 in various manners.

In an embodiment, the roller cover 411 may be positioned on the distal (e.g., the side in the +x direction) of the lower base body 410. For example, a plurality of roller covers 411 may be provided. For example, the number of roller covers 411 may be formed to correspond to the number of roller modules 21 provided in the medical instrument control device 100. Each roller cover 411 may be configured to cover at least a portion of each roller module 21 from the upper side (e.g., the side in the +z direction). For example, the roller cover 411 may include a first roller cover 411a, a second roller cover 411b, a third roller cover 411c, a fourth roller cover 411d, and a fifth roller cover 411e configured to cover the first roller module 21a, the second roller module 21b, the third roller module 21c, the fourth roller module 21d, and the fifth roller module 21e from the upper side (e.g., the side in the +z direction). The first roller cover 411a, the second roller cover 411b, the third roller cover 411c, the fourth roller cover 411d, and the fifth roller cover 411e may be spaced apart from each other in a width direction (e.g., the y direction). The roller cover 411 may be stepped and extend upward (e.g., the side in the +z direction) from the lower base body 410. For example, the roller cover 411 may be substantially integrally formed with the lower base body 410. A space where the roller module 21 is positioned may be formed on the lower side (e.g., the side in the -z direction) of the roller cover 411.

In an embodiment, at least one magnet (not shown) may be disposed on at least a portion of the plurality of roller covers 411. For example, the magnets may be embedded in the second roller cover 411b and the fourth roller cover 411d. However, this is an example, and the position and/or number of the magnets are not limited thereto.

In an embodiment, a plurality of main valleys 412 may be formed. The plurality of main valleys 412 may extend downward (e.g., the side in the -z direction) between the plurality of roller covers 411. For example, each main valley 412 may extend downward (e.g., the side in the -z direction) between two adjacent roller covers 411. The main valley 412 may be substantially formed in a V or U shape. The main valley 412 may form at least some sections of the channel C. For example, the channel of which the upper portion (e.g., the portion in the +z direction) is open may be formed on a valley portion of the main valley 412 to place the medical instrument T thereon. While the roller cover 411 is disposed to cover the upper side (e.g., the side in the +z direction) of the roller module 21, the plurality of main valleys 412 may be positioned between the respective roller modules 21. For example, the main valley 412 may include a first main valley 412a, a second main valley 412b, a third main valley 412c, and a fourth main valley 412d.

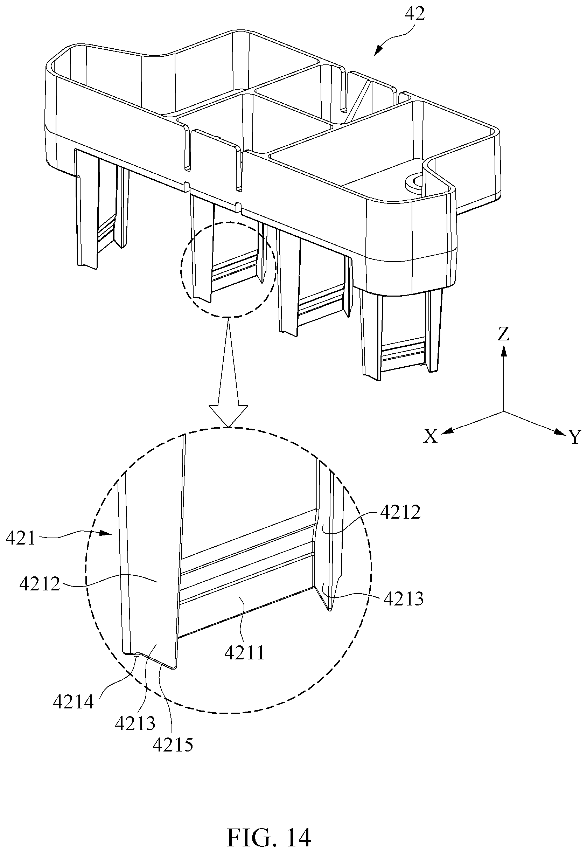

In an embodiment, the main valley 412 may include a valley body 4121 and a support bump 4122 (refer to FIG. 9). The valley body 4121 may be substantially formed in a valley shape. For example, the valley body 4121 may be substantially formed in a V or U shape. The valley body 4121 may form at least some sections of the channel C. A blade (e.g., a blade 4211 of FIG. 14) of the upper guide 42 described below may be inserted into the valley body 4121. The support bump 4122 may protrude in a distal direction (e.g., the +x direction) and/or a proximal direction (e.g., the -x direction) from a lower end (e.g., an end in the -z direction) of the valley body 4121. A pillar (e.g., a pillar 4212 of FIG. 14) and a toe (e.g., a toe 4213 of FIG. 14) of the upper guide 42 described below may be placed on the support bump 4122 and may be supported by the support bump 4122. The support bump 4122 may form a portion of the channel C of which the upper portion (e.g., the +z direction) is open. The support bump 4122 may include a central space 4123 and an inclined wall 4124. The central space 4123 may be a space for forming the channel C. The inclined walls 4124 may be formed on both sides (e.g., the sides in the +y direction and the -y direction) of the central space 4123 and may be incline downward (e.g., the -z direction) from the center of the central space 4123 toward the both sides. The support bump 4122 may be formed to have a width (e.g., the width in the y direction) that narrows toward the distal direction (e.g., the +x direction) or the proximal direction (e.g., the -x direction). Alternatively, the support bump 4122 may be formed to have a substantially constant width (e.g., the width in the y direction). However, this is an example, and the shape of the valley body 4121 and/or the support bump 4122 is not limited thereto.

In an embodiment, the recessed valley 413 may be recessed from the lower base body 410. The recessed valley 413 may form the channel to dispose the medical instrument T together with the main valley 412. A plurality of recessed valleys 413 may be formed. For example, the recessed valley 413 may be formed in a number corresponding to the number of main valleys 412. For example, the recessed valley 413 may include a first recessed valley 413a, a second recessed valley 413b, a third recessed valley 413c, and a fourth recessed valley 413d. The first recessed valley 413a, the second recessed valley 413b, the third recessed valley 413c, and the fourth recessed valley 413d may be substantially formed on an extended line of the first main valley 412a, the second main valley 412b, the third main valley 412c, and the fourth main valley 412d, respectively. The first recessed valley 413a, the second recessed valley 413b, the third recessed valley 413c, and the fourth recessed valley 413d may form a first channel Ca, a second channel Cb, a third channel Cc, and a fourth channel Cd together with the first main valley 412a, the second main valley 412b, the third main valley 412c, and the fourth main valley 412d, respectively. At least some of the plurality of channels C may be substantially formed in a straight shape. For example, the second channel Cb and the third channel Cc may be substantially formed in a straight shape. For example, the first channel Ca and the fourth channel Cd may be formed in a curved shape. However, this is an example and the shapes of the first channel Ca, the second channel Cb, the third channel Cc, and the fourth channel Cd are not limited thereto.

In an embodiment, the recessed valley 413 may include a first recessed space 4131 and a second recessed space 4132 (refer to FIG. 10). The first recessed space 4131 may be a space of which the width narrows toward the lower side (e.g., the -z direction). For example, the first recessed space 4131 may have a triangular cross-sectional shape of which the width substantially narrows toward the lower side (e.g., the -z direction). The second recessed space 4132 may be positioned lower (e.g., the -z direction) than the first recessed space 4131 and may be connected to the first recessed space 4131. The second recessed space 4132 may be a space of which the width remains substantially the same or narrows relatively gradually compared to the first recessed space 4131. For example, the second recessed space 4132 may have a substantially rectangular or trapezoidal cross-sectional shape. Since the width of the upper portion (e.g., the portion in the +z direction) of the first recessed space 4131 is relatively wide, user convenience may be improved when the user inserts the medical instrument T into the recessed valley 413. In addition, since the width of the first recessed space 4131 narrows toward the lower side (e.g., the - z direction), the medical instrument T inserted into the upper portion (e.g., the portion in the +z direction) of the first recessed space 4131 may be naturally guided and placed on the second recessed space 4132. During the procedure, the medical instrument T may move forward/backward and/or rotate in the second recessed space 4132. Since the second recessed space 4132 is formed with the substantially uniform width, the frequency of the medical instrument T contacting an inner wall of the recessed valley 413 may be reduced while the medical instrument T moves forward/backward and/or rotates in the second recessed space 4132.

In an embodiment, the expansion groove 414 may be recessed from the lower base body 410 to have a wider width (e.g., the width in the y direction) than the recessed valley 413 at a proximal end (e.g., the end in the -x direction) of the recessed valley 413. The expansion groove 414 may be substantially formed at a proximal end (e.g., the end in the -x direction) of the lower base body 410. For example, the expansion groove 414 may be formed to have a wider width (e.g., the width in the y direction) toward the proximal direction (e.g., the -x direction). A plurality of expansion grooves 414 may be formed. For example, the expansion groove 414 may be formed in a number corresponding to the number of recessed valleys 413. For example, the expansion groove 414 may include a first expansion groove 414a, a second expansion groove 414b, a third expansion groove 414c, and a fourth expansion groove 414d. The first expansion groove 414a, the second expansion groove 414b, the third expansion groove 414c, and the fourth expansion groove 414d may be respectively formed on the proximal end (e.g., the end in the -x direction) of each of the first recessed valley 413a, the second recessed valley 413b, the third recessed valley 414c, and the fourth recessed valley 413d. The expansion groove 414 may improve the convenience for the user in placing the medical instrument T on the recessed valley 413 by expanding the width (e.g., the width in the y direction) of a proximal end (e.g., the end in the -x direction) of the channel C.

In an embodiment, the holder disposition hole 415 may penetrate the lower base body 410. The holder disposition hole 415 may provide a space to dispose a front holder (e.g., the front holder 6 of FIG. 3). The holder disposition hole 415 may be formed at a position of at least a portion of the plurality of recessed valleys 413. For example, one or a plurality of holder disposition holes 415 may be formed. For example, a first holder disposition hole 415a and a second holder disposition hole 415b may be formed on some sections of the second recessed valley 413b and the third recessed valley 413c, respectively. However, this is an example, and the position and/or number of the holder disposition holes 415 is not limited thereto. The front holder 6 may be exposed upward (e.g., the side in the +z direction) through the holder disposition hole 415 and may hold the medical instrument T positioned on the corresponding recessed valley 413.

In an embodiment, the expanded space 416 may be formed on a proximal side (e.g., the side in the -x direction) of the main valley 412. The expanded space 416 may be formed with a wider width (e.g., the width in the y direction) than the main valley 412 between the roller cover 411 and the lower base body 410. The expanded space 416 may be substantially formed in a space for connecting the roller cover 411 to the lower base body 410. A plurality of expanded spaces 416 may be formed. For example, the expanded space 416 may include a first expanded space 416a, a second expanded space 416b, a third expanded space 416c, and a fourth expanded space 416d. For example, the first expanded space 416a and the fourth expanded space 416d may be substantially adjacent to the first main valley 412a and the fourth main valley 412d, respectively. For example, the second expanded space 416b and the third expanded space 416c may be spaced apart from the second main valley 412b and the third main valley 412c in the proximal direction (e.g., the -x direction), respectively. However, this is an example, and the position, number, and/or shape of the expanded space 416 are not limited thereto. As the expanded space 416 is formed with the wider width (e.g., the width in the y direction) than the main valley 412, the convenience for the user in inserting the medical instrument T into the main valley 412 may be improved. For example, in a medical instrument such as a balloon catheter, an adapter (e.g., a lure lock) may be attached to an end thereof to connect an apparatus for balloon dilatation. As the expanded space 416 is formed with the wider width (e.g., the width in the y direction) than the main valley 412 in the proximal side (e.g., the side in the -x direction) of the main valley 412, the adapter described above may be positioned in the expanded space 416 when the medical instrument (e.g., a balloon catheter) is positioned on the main valley 412. However, this is an example, and a component positioned in the expanded space 416 is not limited thereto.

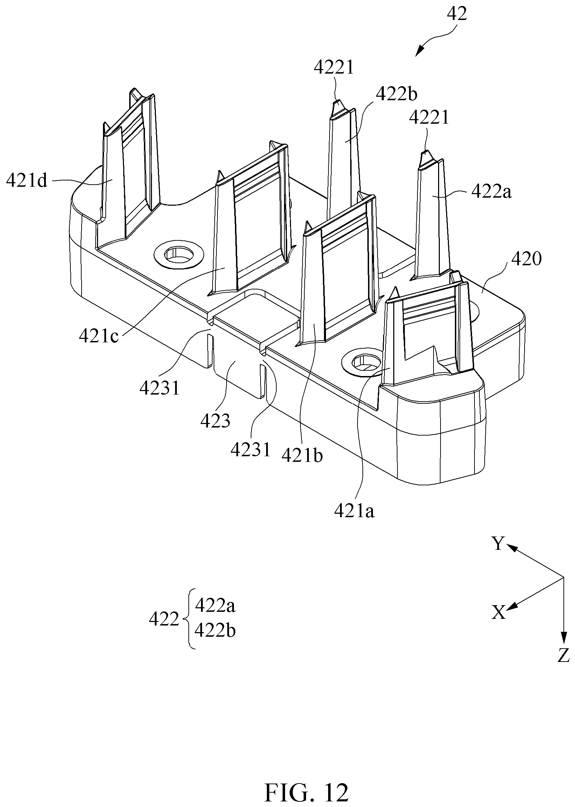

In an embodiment, the additional support bump 417 may protrude in the distal direction (e.g., the +x direction) at a distal end (e.g., the end in the +x direction) of at least a portion of the plurality of recessed valleys 413 (refer to FIG. 7). The additional support bump 417 may be spaced apart from the main valley 412 in the proximal direction (e.g., the -x direction). A space in which two adjacent roller modules 21 are connected to each other may be formed between the main valley 412 and the additional support bump 417. The two adjacent roller modules 21 may be in contact with the medical instrument T positioned in the corresponding channel C through the space between the main valley 412 and the additional support bump 417. One or a plurality of additional support bumps 417 may be formed. For example, the additional support bump 417 may include a first additional support bump 417a and a second additional support bump 417b. The first additional support bump 417a may protrude in the distal direction (e.g., the +x direction) from a distal end (e.g., the end in the +x direction) of the second recessed valley 413b. The second additional support bump 417b may protrude in the distal direction (e.g., the +x direction) from a distal end (e.g., the end in the +x direction) of the third recessed valley 413c. The additional support bump 417 may substantially form a portion of the channel C. A toe (e.g., a toe 4221 of FIG. 12) of an additional leg (e.g., an additional leg 422 of FIG. 12) of the upper guide 42 described below may be placed on the additional support bump 417 and may be supported. The additional support bump 417 may be formed in a shape substantially the same as the support bump 4122 described above. The description of the support bump 4122 described above applies to a detailed description of the shape of the additional support bump 417.

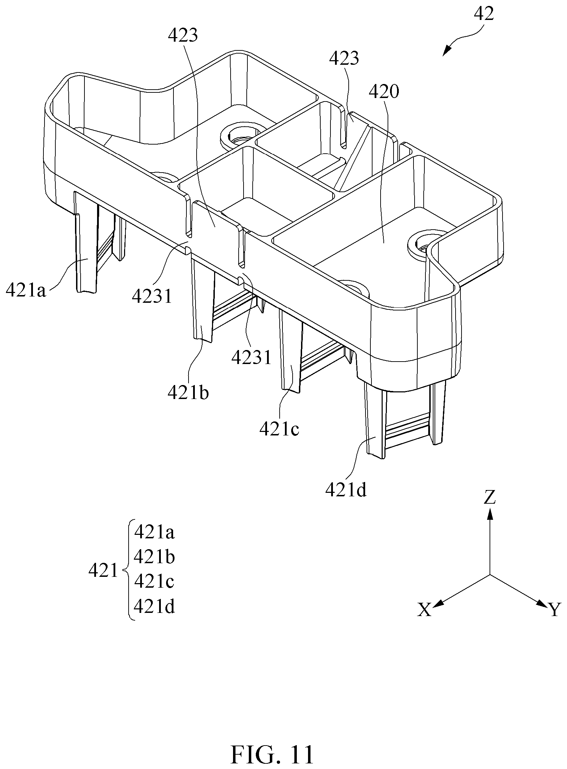

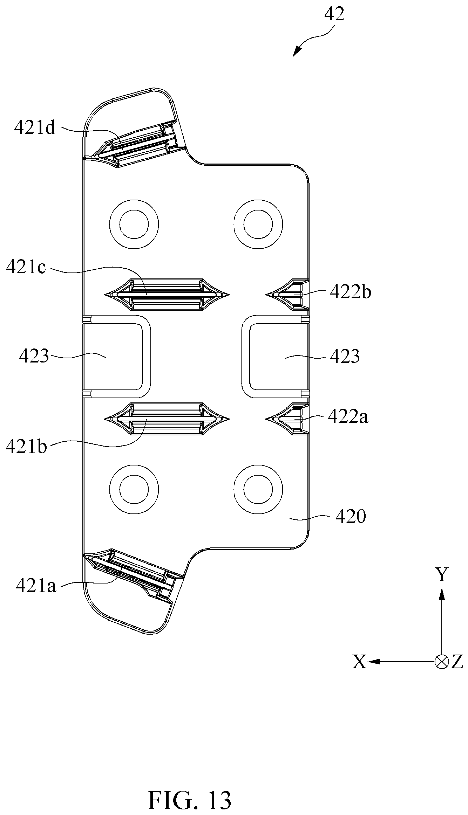

FIG. 11 is a perspective view of an upper guide according to an embodiment. FIG. 12 is a bottom perspective view of an upper guide according to an embodiment. FIG. 13 is a bottom view of an upper guide according to an embodiment. FIG. 14 is a perspective view of a leg according to an embodiment.

Hereinafter, the upper guide 42 is described with reference to FIGS. 1 to 14. Hereinafter, in the description of the upper guide 42, it may be construed that proximal may refer to a side in the -x direction and distal may refer to a side in the +x direction.

In an embodiment, the upper guide 42 may include an upper base body 420, a leg 421, an additional leg 422, and an detachment assist 423.

In an embodiment, the upper base body 420 may form a base of the upper guide 42. The upper base body 420 may be placed on the upper side (e.g., the side in the +z direction) of the plurality of roller covers 411. At least one magnet (not shown) may be disposed on the upper base body 420. For example, the magnet positioned on the upper base body 420 may be formed at a position corresponding to a magnet positioned on the roller cover 411 of the lower guide 41. The upper base body 420 and the roller cover 411 may be attached to and detached from each other by the magnet. In other words, the upper guide 42 and the lower guide 41 may be coupled to each other by magnetic force.

In an embodiment, the leg 421 may extend downward (e.g., the -z direction) from a lower portion (e.g., a portion in the -z direction) of the upper base body 420. A plurality of legs 421 may be formed. For example, the leg 421 may be formed in a number corresponding to the number of main valleys 412. While the upper guide 42 is connected to the lower guide 41, each leg 421 may be inserted into the corresponding main valley 412. The leg 421 may be substantially formed in a shape corresponding to the main valley 412. When the leg 421 is inserted into the main valley 412, each leg 421 may close the upper portion (e.g., the portion in the +z direction) of the channel C formed on the respective main valley 412. For example, the leg 421 may include a first leg 421a, a second leg 421b, a third leg 421c, and a fourth leg 421d. The first leg 421a, the second leg 421b, the third leg 421c, and the fourth leg 421d may be inserted into the first main valley 412a, the second main valley 412b, the third main valley 412c, and the fourth main valley 412d, respectively.

In an embodiment, the leg 421 may include a blade 4211, a pillar 4212, and a toe 4213 (refer to FIG. 14). The blade 4211 may be substantially formed in a shape corresponding to the valley body 4121. The blade 4211 may be inserted into the valley body 4121 and may close the upper portion (e.g., the portion in the +z direction) of the channel C formed on the valley body 4211. For this, a length (e.g., the length in the z direction) of the blade 4211 may be less than a depth (e.g., the depth in the z direction) of the valley body 4121. The pillar 4212 may be formed to have a wider width (e.g., the width in the y direction) than the blade 4211 at a distal end (e.g., the end in the +x direction) and/or a proximal end (e.g., the end in the -x direction) of the blade 4211. For example, the pillar 4212 may be substantially formed in a pillar shape. While the blade 4211 is inserted into the valley body 4121, the pillar 4212 may be positioned on a distal side (e.g., the side in the +x direction) and/or a proximal side (e.g., the side in the -x direction) of the valley body 4121. According to the structure described above, the leg 421 may be stably inserted into the main valley 412 and may be positioned. The toe 4213 may be formed on a lower end (e.g., the end in the -z direction) of the pillar 4212. The toe 4213 may be substantially formed in a shape corresponding to the support bump 4122. While the blade 4211 is inserted into the valley body 4121, the toe 4213 may be placed on the support bump 4122. The toe 4213 may include a central space 4214 and an inclined wall 4215. The central space 4214 may be a space for forming the channel C. The inclined wall 4215 may be formed to incline downward (e.g., the -z direction) at both sides (e.g., the sides in the +y direction and the -y direction) of the central space 4214 from the center toward the both sides. The toe 4213 may be formed to have a width (e.g., the width in the y direction) that narrows toward the distal direction (e.g., the +x direction) or the proximal direction (e.g., the -x direction). Alternatively, the toe 4213 may be formed to have a substantially constant width (e.g., the width in the y direction). However, this is an example, and the shapes of the blade 4211, the pillar 4212, and/or the toe 4213 are not limited thereto.

In an embodiment, the additional leg 422 may extend downward (e.g., the -z direction) from a lower portion (e.g., a portion in the -z direction) of the upper base body 420. For example, the additional leg 422 may be placed on the additional support bump 417 through the expanded space 416. A plurality of additional legs 422 may be formed. For example, the additional leg 422 may be formed in a number corresponding to the number of additional support bumps 417. For example, the additional leg 422 may include a first additional leg 422a and a second additional leg 422b. The first additional leg 422a and the second additional leg 422b may be formed at positions corresponding to the first additional support bump 417a and the second additional support bump 417b and may be placed on the first additional support bump 417a and the second additional support bump 417b. The first additional leg 422a and the second additional leg 422b may be spaced apart from the second main leg 421b and the third main leg 421c in the proximal direction (e.g., the -x direction). A space in which two adjacent roller modules 21 are connected to each other may be formed between the additional leg 422 and the leg 421. The two adjacent roller modules 21 may be in contact with the medical instrument T positioned in the corresponding channel C through the space between the additional leg 422 and the leg 421.

In an embodiment, a toe 4221 may be formed at a lower end (e.g., the end in the -z direction) of the additional leg 422 (refer to FIG. 12). The toe 4221 of the additional leg 422 may be substantially formed in a shape corresponding to the additional support bump 417. The toe 4221 of the additional leg 422 may be formed in a shape substantially the same as the toe 4213 of the leg 421 described above. The description of the toe 4213 of the leg 421 described above applies to a detailed description of the shape of the toe 4221 of the additional leg 422.

In an embodiment, the detachment assist 423 may be a component that helps decoupling of magnetic coupling between the lower guide 41 and the upper guide 42. For example, the detachment assist 423 may be formed as a pair at positions facing each other. The detachment assist 423 may be formed by cutting a portion of a side surface (e.g., the side surface in the +x/-x direction) and a bottom surface (e.g., the surface in the -z direction) of the upper base body 420. For example, the detachment assist 423 may be cut except for a shaft portion 4231 and may be substantially connected to the upper base body 420 through the shaft portion 4231. The detachment assist 423 may elastically rotate relative to the upper base body 420 based on the shaft portion 4231. For example, while the lower guide 41 and the upper guide 42 are coupled to each other by the magnet, the user may press the side surface (e.g., the side surface in the +x/-x direction) of the detachment assist 423 to decouple the magnetic coupling between the lower guide 41 and the upper guide 42. When the side surface (e.g., the side surface in the +x/-x direction) of the detachment assist 423 is pressed, the bottom surface (e.g., the surface in the -z direction) of the detachment assist 423 may be pressed downward (e.g., the -z direction) as the detachment assist 423 elastically rotates relative to the upper base body 420 based on the shaft portion 4231. Accordingly, as the bottom surface (e.g., the surface in the -z direction) of the detachment assist 423 pushes the lower guide 41, he upper guide 42 may be separated from the lower guide 41 by overcoming the magnetic force between the lower guide 41 and the upper guide 42. According to the structure described above, the convenience for the user in separating the upper guide 42 from the lower guide 41 may be improved.

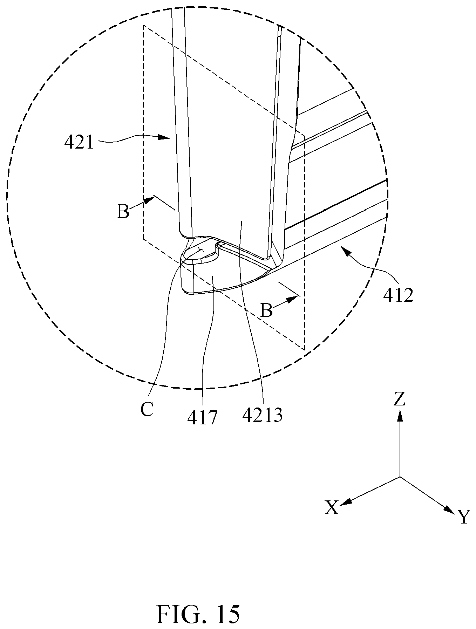

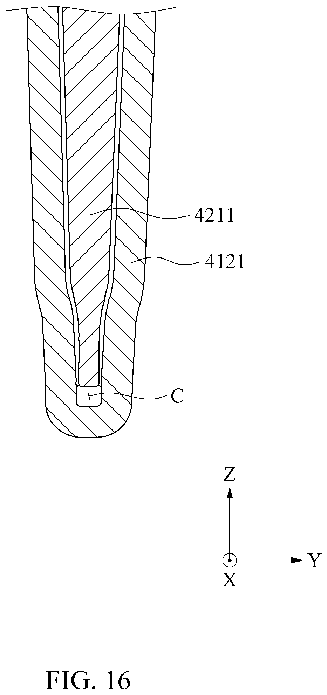

FIG. 15 is an enlarged view of portion A of FIG. 5. FIG. 16 is a cross-sectional view of portion B-B of FIG. 15.

Referring to FIGS. 5, 15, and 16, while the lower guide 41 is connected to the upper guide 42, the leg 421 may be inserted into the main valley 412. The leg 421 may close the upper portion (e.g., the portion in the +z direction) of the channel C formed on the main valley 412. For example, as shown in FIG. 16, the blade 4211 may be formed shorter than the valley body 4121 and may close the upper portion (e.g., the portion in the +z direction) of the channel C. In addition, as shown in FIG. 15, as the toe 4213 of the leg 421 is placed on the support bump 417, the channel C may be formed between the toe 4213 and the support bump 417. The case of an additional support bump (e.g., the additional support bump 417 of FIG. 8) and a toe (e.g., the toe 4221 of FIG. 12) of an additional leg (e.g., the additional leg 422 of FIG. 12) may be understood in the same manner. According to the structure described above, when the upper guide 42 is coupled to the lower guide 41 while a medical instrument (e.g., the medical instrument T of FIG. 3) is placed on the channel C formed on the lower guide 41, in at least a portion (e.g., at least the portion of the main valley 412 and/or the additional support bump 417) of the channel C, the upper portion (e.g., the portion in the +z direction) of the channel C may be closed. Accordingly, the medical instrument T may be stably positioned in the channel C and the path thereof may be stably guided along the channel C.

Although the embodiments have been described with reference to the limited drawings, one of ordinary skill in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A medical instrument guide connected to a medical instrument control device, the medical instrument guide comprising:
a lower guide in which a plurality of channels with open upper portions is formed to dispose a medical instrument thereon; and
an upper guide connected to an upper side of the lower guide to close an upper portion of at least a section of each of the channels.

2. The medical instrument guide of claim 1,
wherein the lower guide comprises:
a lower base body;
a plurality of roller covers positioned on a distal of the lower base body to cover at least a portion of a plurality of roller modules provided in the medical instrument control device from an upper portion; and
a plurality of main valleys forming at least a section of the channel and extending downward between the plurality of roller covers to be positioned between each two of the plurality of roller modules.

3. The medical instrument guide of claim 2,
wherein the upper guide comprises:
an upper base body placed on an upper side of the plurality of roller covers; and
a plurality of legs extending downward from a lower portion of the upper base body to be inserted into each of the plurality of main valleys.

4. The medical instrument guide of claim 3,
wherein, while the upper guide is connected to the lower guide, the plurality of legs closes the upper portion of each of the channels formed on the plurality of main valleys.

5. The medical instrument guide of claim 3,
wherein each of the main valleys comprises:
a valley body forming at least a section of the channel; and
a support bump protruding in a distal direction or a proximal direction at a lower end of the valley body.

6. The medical instrument guide of claim 5,
wherein each of the legs comprises:
a blade that is inserted into the valley body and closes the upper portion of the channel formed on the valley body;
a pillar formed to have a width that is wider than the blade at a distal end or a proximal end of the blade; and
a toe formed in a shape corresponding to the support bump at a lower end of the pillar.

7. The medical instrument guide of claim 6,
wherein the support bump and the toe each comprise:
a central space for forming the channel; and
inclined walls formed on both sides of the central space and inclined downward from a center of the central space toward the both sides.

8. The medical instrument guide of claim 3,
wherein the lower guide further comprises:
a plurality of expanded spaces formed with a width that is greater than the main valley between the roller cover and the lower base body on a proximal side of each of the plurality of main valleys.

9. The medical instrument guide of claim 3,
wherein the lower guide further comprises:
a plurality of recessed valleys recessed from the lower base body to form the channel together with the plurality of main valleys.

10. The medical instrument guide of claim 9,
wherein the lower guide further comprises:
a plurality of expansion grooves recessed from the lower base body to have a wider width than the recessed valley at a proximal end of each of the plurality of recessed valleys.

11. The medical instrument guide of claim 9,
wherein the lower guide further comprises:
a holder disposition hole penetrating the lower base body at a position of at least a portion of the plurality of recessed valleys to dispose a holder for holding a medical instrument positioned on the recessed valley.

12. The medical instrument guide of claim 9,
wherein the lower guide further comprises an additional support bump protruding from a distal end of at least a portion of the plurality of recessed valleys in a distal direction, and
the upper guide further comprises an additional leg extending downward from a lower portion of the upper base body to be placed on the additional support bump.

13. The medical instrument guide of claim 9,
wherein each of the plurality of recessed valleys comprises:
a first recessed space of which a width narrows toward a lower side; and
a second recessed space of which a width remains the same at a lower side compared to the first recessed space.

14. The medical instrument guide of claim 3,
wherein at least one magnet is disposed on the plurality of roller covers and at least one magnet is disposed on the upper base body to couple the lower guide and the upper guide to each other by a magnetic force.

15. The medical instrument guide of claim 14,
wherein the upper guide further comprises:
a detachment assist formed by cutting a portion of a side surface and a bottom surface of the upper base body to elastically rotate relative to the upper base body to help decoupling of magnetic coupling between the lower guide and the upper guide.
